Europäisches Patentamt

European Patent Office

Office européen des brevets

(19)

(11) Publication number: **0 084 342**
**B1**

(12) **EUROPEAN PATENT SPECIFICATION**

(45) Date of publication of patent specification: **07.01.87**

(51) Int. Cl.⁴: **B 08 B 3/02, B 05 B 3/02**

(21) Application number: **83100183.9**

(22) Date of filing: **11.01.83**

(54) Washing apparatus.

(30) Priority: **19.01.82 JP 6575/82**

(43) Date of publication of application:
**27.07.83 Bulletin 83/30**

(45) Publication of the grant of the patent:
**07.01.87 Bulletin 87/02**

(84) Designated Contracting States:
**AT BE CH DE FR GB IT LI NL SE**

(56) References cited:
**DE-B-1 131 372**
**US-A-3 974 843**
**US-A-4 132 567**

(73) Proprietor: **OLYMPUS OPTICAL CO., LTD.**
**43-2, 2-chome, Hatagaya Shibuya-ku**
**Tokyo 151 (JP)**

(72) Inventor: **Ogasawara, Tadahiko**
**Olympus-Ishikawa-ryo 2544 Ishikawa-machi**
**Hachioji-shi Tokyo (JP)**

(74) Representative: **Kuhnen, Wacker & Partner**
**Schneggstrasse 3-5 Postfach 1729**
**D-8050 Freising (DE)**

Courier Press, Leamington Spa, England.

## Description

The present invention relates to a washing apparatus according to the preamble of claim 1.

In washing, an object such as an endoscope, for example, in a washing tank, a washing apparatus in which a washing nozzle is rotated for improved washing effect has been conventionally used.

US—PS 4,132,567 shows an apparatus for cleaning and removing static charges from substrates, for example semiconductor wavers. This apparatus is an example for prior art washing apparatuses, which use a washing nozzle which is rotated by connecting the supporting shaft bearing the nozzle directly to a motor or via a suitable gear as a drive source. Namely, the supporting shaft is rotatably past through the bottom portion of the washing tank in a liquid-tight manner with the aid of a sealing member. The washing nozzle is attached to the upper end portion of the supporting shaft which projects into the washing tank, and the lower end portion of the supporting shaft protruding from the washing tank is coupled with the rotating shaft of the motor.

In such an arrangement, however, a minimum of eccentricity between the supporting shaft and the motor shaft will substantially deteriorate the sealing efficiency and durability of the sealing member. It is therefore essential to align the axes of these two shafts with high accuracy. Accordingly, manufacture and assembly require high-accuracy operations, resulting in reduced productivity and increased cost. At the rotating sealing portion, moreover, the sealing member is liable to be damaged early and its sealing efficiency may be reduced.

It is therefore an object of the present invention to provide the washing apparatus wherein the necessity of high-accuracy operations for working and assembly can be obviated and improved sealing effects are insured.

Solution of this object is achieved by the characterizing features of claim 1.

According to claim 1 there is provided a rotating shaft being disposed outside the housing and being coaxial with a supporting shaft carrying the washing nozzle, wherein the rotating shaft is coupled to the supporting shaft via magnetic coupling members, so that the rotational torque of the rotating shaft is transmitted by means of magnetic forces through the bottom wall of the washing chamber to the supporting shaft.

By means of this no problems with respect to provide a sealing relationship between the rotating shaft and the bottom wall of the washing chamber and no problems with respect to possible disalignments between supporting shaft and rotating shaft do occur.

The use of a magnetic coupling means is per se known from DE—B—1 131 372, but this known device is used for a kitchen-pot, and the problems with respect to a sealing member between a rotating shaft and a wall of a container are still inherent with this device.

This invention can be more fully understood from the following detailed description when taken in conjunction with the accompanying drawings, in which:

Fig. 1 is a sectional view of a washing apparatus according to an embodiment of this invention; and

Fig. 2 is a sectional view showing modified magnetic coupling members of the washing apparatus.

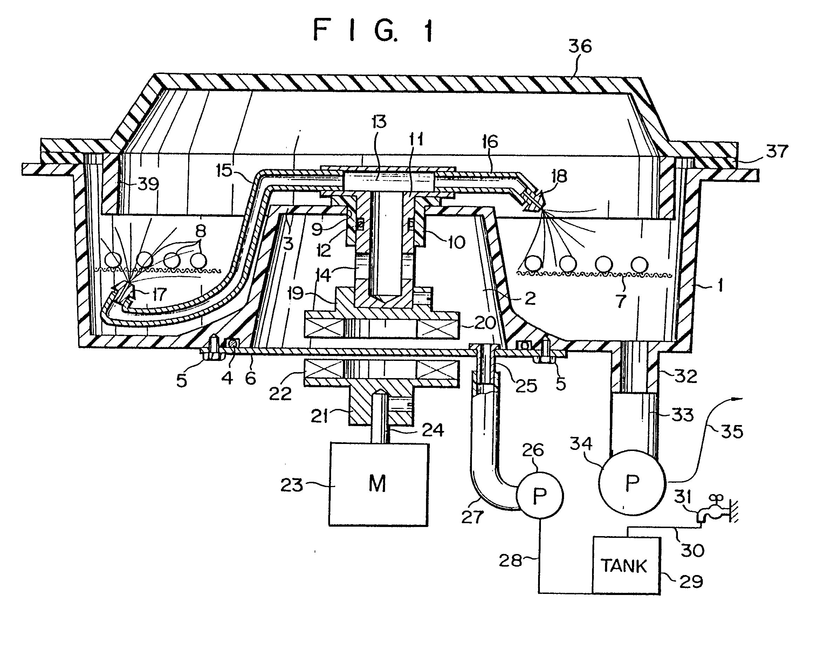

Referring now to Fig. 1, an embodiment of this invention will be described in detail. In Fig. 1, numeral 1 designates a washing tank open at the top and having a washing chamber therein. A cylindrical projection 3 protrudes upwards from the bottom of the washing tank 1 into the cavity of the washing tank 1, defining a liquid reservoir portion 2 therein. The bottom opening of the projection 3 is closed in a liquid-tight manner with a partition wall 6 of a nonmagnetic material which is attached to the bottom of the washing tank 1 by means of screws 5 with a gasket 4 interposed between them. The partition wall 6 and the washing tank 1 constitute a housing. A holder net 7 is stretched horizontally around the projection 3 in the washing tank 1. Endoscopes 8 as object to be washed are put on the net 7 with their insertion sections rolled up.

A circular mounting hole 9 is bored through the center of the top wall of the projection 3, and a cylindrical bearing 10 is fitted in the mounting hole 9. A supporting shaft 11 penetrates the bearing 10 to be rotatably supported thereby in a liquid-tight manner with the aid of a packing 12. The supporting shaft 11 has a thick portion located above the projection 3 and a slender portion protruding downward from the thick portion to be located inside the projection 3. The supporting shaft 11 is provided with a T-shaped passageway 13 which is formed of a horizontal hole bored through the thick portion and a vertical hole bored through the slender portion. Also, an intake hole 14 connecting the passageway 13 and the reservoir portion 2 is bored through the peripheral wall of the supporting shaft 11 near the lower end portion thereof which projects into the reservoir portion 2. First and second nozzle guides 15 and 16 opening into the passageway 13 are coupled to the thick portion of the supporting shaft 11 so that one end of each nozzle guide is inserted into each corresponding end of the horizontal hole. The other end portion of the first nozzle guide 15 is led under the holder net 7, and a first washing nozzle 17 is attached to the extreme end of the guide 15 facing upward. The other end portion of the second nozzle guide 16 is led over the holder net 7, and a second washing nozzle 18 is attached to the extreme end of the guide 16 facing downward. A driven-side yoke 19 is coaxially mounted on the outer surface of the lower end portion of the supporting shaft 11. A first permanent magnet (magnetic coupling member) 20 is attached to the under surface of the driven-side yoke 19 facing the partition wall 6 so that the magnet 20 and the partition wall 6 are arranged parallel with a narrow space between them. A second permanent magnet (magnetic coupling member) 22 is attached to a driving-side yoke 21 to be magnetically coupled with the first magnet 20. The second magnet 22 is located in that position under the washing tank 1 which faces the first magnet 20 through the partition wall 6 so that the magnet 22 and the partition wall 6 are arranged parallel with a narrow space between them.

The first magnet 20 and the second magnet 22 are both ring-shaped and magnetically coupled to each other. Each of them has a plurality of N and S poles alternately arranged in the circumferential direction, preferably two N poles and two S poles or four N poles and four S poles. The driving-side yoke 21 to which the magnet 22 is attached is coaxially connected to the shaft 24 of a motor 23. When the motor 23 is driven, the second magnet 22 rotates the first magnet 20, whereby the supporting shaft 19 is rotated.

A supply port pipe 25 is disposed through the partition wall 6. The lower end of the pipe 25 is connected to one end of a supply tube 27. The other end of the tube 27 is connected to the outlet port of a supply pump 26 which is provided outside the washing tank 1. The inlet port of the pump 26 is connected to a storage tank 29 via a tube 28. The storage tank 29 is connected via a tube 30 to a faucet 31. A discharge port pipe 32 is disposed through the bottom of the washing tank 1. The lower end of the pipe 32 is connected via a discharge tube 33 to the inlet port of a discharge pump 34. The outlet port of the discharge pump 34 is connected to one end of a tube 35. The other end of the tube 35 is connected to a tank (not shown) for storing the liquid discharged from the washing tank 1.

A cover 36 is detachably mounted on the top of the washing tank 1 in a liquid-tight manner with the aid of a gasket 37 so that the top of the washing tank 1 can be opened. An annular screening plate 39 protrudes from the peripheral portion of the inside of the cover 36. As will be mentioned later, the screening plate 39 prevents a liquid jetting from the first and second washing nozzles 17 and 18 from leaking through the junction of the washing tank 1 and the cover 36.

There will now be described the operation of the apparatus with the aforementioned construction. First, a liquid feeding pump 26 is actuated to pressurize tap water stored in the liquid tank 29 and to supply it to the liquid reservoir portion 2. The tap water supplied to the liquid reservoir portion 2 flows into the passageway 13 of the supporting shaft 11 through the intake hole 14. Then, the water passes through the first and second nozzle guides 15 and 16 connected to the passageway 13 to be ejected from the first and second washing nozzles 17 and 18. At the same time that the liquid feeding pump 26 starts, a motor 23 is activated, and the second magnet 22 is rotated as a rotating shaft 24 of the motor 23 rotates. Then, the first magnet 20 magnetically coupled with the second magnet 22 across the nonmagnetic partition wall 6 rotates together with the second magnet 22, so that the supporting shaft 11, united with the first magnet 20, is rotated. As the supporting shaft 11 rotates, the first and second washing nozzles 17 and 18, which are supported on the supporting shaft 11 by means of the first and second nozzle guides 15 and 16, also rotate. The water jetted from the washing nozzles 17 and 18 is uniformly sprayed on the endoscopes 8 on the holder net 7 from both upper and lower sides. Thus, the endoscopes 8 are satisfactorily washed by a spray of pressurized tap water. The water collected in the washing tank 1 after spraying is discharged into a drainage tank (not shown) by a drainage pump 34.

According to the washing apparatus thus constructed, the supporting shaft 11 and the rotating shaft 24 of the motor 23 are linked together as the first and second magnets 20 and 22 thereon are magnetically coupled across the partition wall 6. Accordingly, even though the axes of the shafts 11 and 24 are more or less out of alignment, the supporting shaft 11 can be rotated without strain. Moreover, since the shafts 11 and 24 can be spaced apart, the partition wall 6 can be interposed between them to ensure the liquid-tightness of the liquid reservoir portion 2.

In this construction, even if the water supplied to the liquid reservoir portion 2 leaks out into the washing tank 1 through the gap between the supporting shaft 11 and the bearing 10 supporting the shaft 11, it will never drop onto the motor 23 or other member to damage the same.

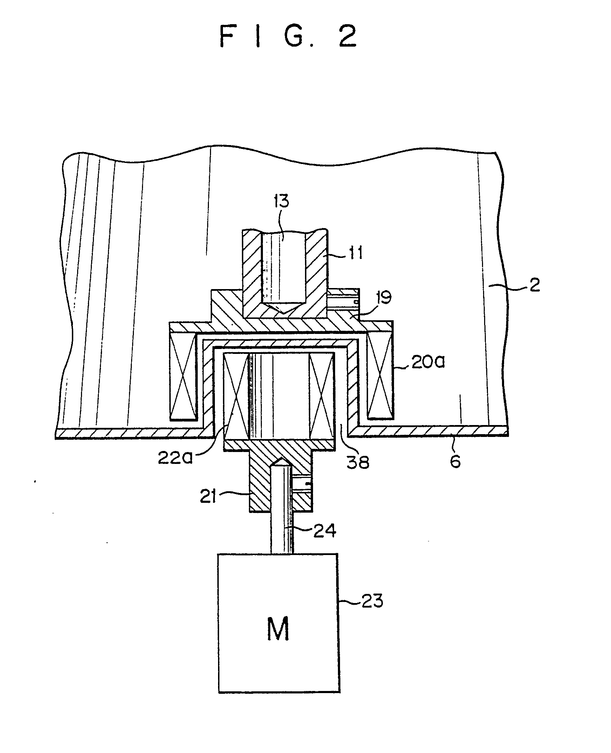

Fig. 2 shows another embodiment of this invention, in which a hollow projection 38 protrudes from the central portion of the partition wall 6 into the liquid reservoir portion 2. Also, a cylindrical first magnet 20a surrounding the projection 38 is attached to the driven-side yoke 19 at the lower end of the supporting shaft 11. The driving-side yoke 21 fixed on the rotating shaft 24 of the motor 23 is fitted with a cylindrical second magnet 22a which is housed in the projection 38 so that its outer peripheral surface faces the inner peripheral surface of the first magnet 20a with the peripheral wall of the projection 38 between them. Also in this construction, the supporting shaft 11 and the rotating shaft 24 of the motor 23 can be linked together, as the first and second magnets 20a and 22a are magnetically coupled.

Instead of being a permanent magnet, an electromagnet can be used. Two washing nozzles need not always be used, and a single nozzle will do. Preferably, however, a plurality of washing nozzles should be provided in order that the endoscope may be washed from both upper and lower sides, as in the aforementioned embodiment.

According to this invention, as described above, a first magnetic coupling member (magnet) is attached to a supporting shaft supporting washing nozzles inside a housing, and a second magnetic coupling member (magnet) is disposed outside the housing so as to be rotated by a drive mechanism. Thus, the supporting shaft can be linked to the drive mechanism through the magnetic coupling between the first and second magnetic coupling members. Although the axes of the supporting shaft and the drive mechanism are not exactly in alignment, the supporting shaft will never be subjected to any unreasonable force, so that manufacture and assembly can be facilitated. Since the supporting shaft and the drive mechanism are coupled in a non-contact manner, it is unnecessary to rotatably pass the supporting shaft through a partition wall between the shaft and the mechanism with the aid of a sealing member. Thus, unlike the prior art apparatus, the washing apparatus of the invention will not suffer leakage after only a short operating life.

## Claims

1. A washing apparatus using a washing fluid, comprising a housing (1, 2) including a washing chamber; at least one washing nozzle (17, 18) capable of injecting the washing fluid into the washing chamber; nozzle holding means for supporting the washing nozzle; a drive source (23) capable of rotating the holding means, thereby rotating the nozzle, wherein said nozzle holding means includes a supporting shaft (11) provided in the washing chamber; and supporting means (10) for rotatably supporting the supporting shaft (11), characterized by a rotating shaft (24) disposed outside the housing to be coaxial with the supporting shaft (11); and magnetic coupling members (20, 22) mounted individually on the rotating shaft and the supporting shaft and capable of magnetically coupling the shafts.

2. The washing apparatus according to claim 1, characterised in that said housing includes a washing tank (1) having an inwardly protruding hollow projection (3) and the washing chamber defined therein, and a partition wall (6) attached to the washing tank to seal the hollow of the projection, said supporting shaft (11) being disposed in the hollow so that the magnetic coupling members (20, 22) individually mounted on the supporting shaft (11) and the rotating shaft (24) face each other with the partition wall therebetween.

3. The washing apparatus according to claim 2, characterized in that said partition wall (6) is formed of a nonmagnetic material.

4. The washing apparatus according to claims 1, 2 or 3, wherein each said magnetic coupling member includes a magnet.

5. The washing apparatus according to claim 2, characterised in that said supporting shaft (11) protrudes from the projection (3) into the washing chamber, extending between the hollow of the projection and the washing chamber in a liquid-tight manner so that the washing nozzle is supported on that portion of the supporting shaft which extends into the washing chamber, and said supply means includes an inlet port portion (25) for feeding the washing fluid from the outside of the housing into the hollow of the projection and a passageway (13) formed in the supporting shaft to connect the hollow of the projection and the washing nozzle.

## Patentansprüche

1. Waschvorrichtung, welche ein Waschfluid verwendet, mit einem Gehäuse (1, 2) welches eine Waschkammer aufweist; wenigstens einer Waschdüse (17, 18) welche das Waschfluid in die Waschkammer spritzt; Düsenhaltemittel zur Lagerung der Waschdüse; einer Antriebsquelle (23), welche die Haltemittel drehbar antreibt, so daß die Düse gedreht wird, wobei die Düsenhaltemittel eine Stützwelle (11) in der Waschkammer aufweisen; und mit Lagermitteln (10) zum drehbeweglichen Lagern der Stützwelle (11), gekennzeichnet durch eine Drehwelle (24), die außerhalb des Gehäuses koaxial mit der Stützwelle (11) angeordnet ist; und eine magnetische Kupplungsvorrichtung (20, 22) welche an der Drehwelle bzw. der Stützwelle angeordnet ist und die beiden Wellen magnetisch miteinander verbindet.

2. Waschvorrichtung nach Anspruch 1, dadurch gekennzeichnet, daß das Gehäuse einen Waschtank (1) aufweist, der einen nach innen vorspringenden hohlen Vorsprung (3) aufweist und in welchem die Waschkammer gebildet ist und eine Trennwand (6) aufweist, welche an dem Waschtank derart angeordnet ist, daß sie den Hohlraum des Vorsprunges abdichtet, wobei die Stützwelle (11) in dem Hohlraum derart angeordnet ist, daß die magnetische Kupplungsvorrichtung (20, 22), welche an der Stützwelle (11) bzw. der Drehwelle (24) angeordnet ist, einander gegenüberliegen und durch die Trennwand voneinander getrennt sind.

3. Waschvorrichtung nach Anspruch 2, dadurch gekennzeichnet, daß die Trennwand (6) aus nichtmagnetischem Material gefertigt ist.

4. Waschvorrichtung nach Anspruch 1, 2 oder 3, dadurch gekennzeichnet, daß jede der magnetischen Kupplungsvorrichtung einen Magneten aufweist.

5. Waschvorrichtung nach Anspruch 2, dadurch gekennzeichnet, daß die Stützwelle (11) von dem Vorsprung (3) in die Waschkammer hinein vorspringt, sich zwischen dem Hohlraum des Vorsprunges und der Waschkammer in flüssigkeitsdichter Anlage erstreckt, so daß die Waschdüse an dem Bereich der Stützwelle gelagert ist, der sich in die Waschkammer erstreckt, und daß eine Zufuhrvorrichtung vorgesehen ist, welche einen Einlaßanschlußbereich (25) zum Zuführen des Waschfluides von außen in den Hohlraum des Vorsprunges und einen Durchlaß (13) in der Stützwelle aufweist, um den Hohlraum des Vorsprunges und die Waschdüse miteinander zu verbinden.

## Revendications

1. Appareil de lavage, utilisant un fluide de lavage, comportant un carter (1, 2), comprenant une chambre de lavage, au moins un ajutage de lavage (17, 18) capable d'injecter le fluide de lavage dans la chambre de lavage, un moyen de soutien d'ajutage pour supporter l'ajutage de lavage, une source de force motrice (23) capable de faire tourner ledit moyen de soutien, et, de ce fait, de faire tourner l'ajutage, ledit moyen de soutien d'ajutage comprenant un arbre support (11) disposé dans la chambre de lavage, et un moyen de support (10) pour supporter dans sa rotation l'arbre support (11), caractérisé par un arbre rotatif (24) disposé à l'extérieur dudit carter (1, 2) et de façon coaxiale à l'arbre support (11), et par des éléments d'accouplement magnétiques (20, 22) montés individuellement sur ledit arbre rotatif (24) et l'arbre support (11) et capables d'accoupler magnétiquement les deux arbres.

2. Appareil de lavage selon la revendication 1, caractérisé par le fait que ledit carter comprend une cuve de lavage (1) ayant une saillie creuse (3) s'étendant vers l'intérieur et la chambre de lavage délimitée à l'intérieur de ladite cuve, et une paroi de séparation (6) fixée à la cuve de lavage pour fermer la partie creuse de ladite saillie, ledit arbre support (11) étant disposé dans la partie creuse, de telle sorte que les éléments d'accouplement magnétiques (20, 22) montés individuellement sur l'arbre support (11) et l'arbre rotatif (24) sont disposés en face l'un de l'autre, la paroi de séparation (6) étant interposée entre eux.

3. Appareil de lavage selon la revendication 2, caractérisé par le fait que ladite paroi de séparation est faite d'un matériau non magnétique.

4. Appareil de lavage selon l'une quelconque des revendications 1 à 3, dans lequel chacun desdits éléments d'accouplement magnétiques comporte un aimant.

5. Appareil de lavage selon la revendication 2, caractérisé par le fait que l'arbre support (11) dépasse de la saillie (3) dans la chambre de lavage et s'étend de façon étanche au liquide entre le creux de ladite saillie et la chambre de lavage, de sorte que l'ajutage de lavage est supporté par la partie dudit arbre support (11) qui s'étend dans la chambre de lavage, et que ledit moyen d'alimentation en liquide de lavage comporte un orifice d'admission (25) pour introduire le liquide de lavage depuis l'extérieur dudit carter dans le creux de ladite saillie, et un passage (13) formé dans l'arbre support (11) pour faire communiquer le creux de ladite saillie et l'ajutage de lavage.

# F I G. 1

0 084 342

# F I G. 2